# EUROPÄISCHE PATENTANMELDUNG

(11) **EP 0 619 124 A1**
(43) Veröffentlichungstag der Anmeldung: **12.10.1994**
(21) Anmeldenummer: 94890023.8
(22) Anmeldetag: 28.01.1994
(51) Int. Cl.: A61N 5/04

(54) **Katheter zur Hyperthermie-Behandlung**

(30) Priorität: 03.02.1993 AT 180/93
(71) Anmelder: OTHMAR HANDL Ges.m.b.H., A-1130 Wien (AT)
(72) Erfinder: Burian, Gerhard, Dipl.-Ing., A-2620 Wartmannstetten 172 (AT)
(74) Vertreter: Pinter, Rudolf, Dipl.-Ing.

(57) **Zusammenfassung**

Die über eine Anschlußleitung (1) von außen angespeiste Mikrowellenantenne (2) ist als Faltdipol (3) ausgebildet, welcher in Form einer Doppelwendel (4) isoliert über einen im wesentlichen zylindrischen, leitfähigen Tragekörper (6), der auch unmittelbar vom Endbereich (8) der Abschirmung (9) eines als Anschlußleitung (1) dienenden Koaxialkabels (7) gebildet sein kann, gewickelt ist. Mittels einer Verschiebung des Einspeisepunktes der Anschlußleitung (1) aus der Mitte der Doppelwendel (4) kann der Eingangswiderstand der Mikrowellenantenne (2) leicht dem Wellenwiderstand der Anschlußleitung (1) angepaßt werden, was insgesamt eine stabile Resonanz der Mikrowellenantenne (2) ergibt und Abstrahlungen der Anschlußleitung (1) hintanhält.

## Beschreibung

Die Erfindung betrifft einen Katheter zur Hyperthermie-Behandlung von lebendem Gewebe, insbesonders der Prostata, mit einer über eine Anschlußleitung von außen angespeisten Mikrowellenantenne.

Die Anwendung von Mikrowellen zur gezielten Erwärmung und damit Zerstörung von Tumorgewebe im bzw. am Körper von Menschen und Tieren ist bekannt, wobei gemäß den jeweiligen Gegebenheiten unterschiedliche Antennenformen, Anspeisungen, Betriebsfrequenzen usw., zum Einsatz kommen. Speziell bei unblutig in den Körper des Patienten über geeignete Körperöffnungen einzuführenden Kathetern mit von außen angespeister Mikrowellenantenne sind natürlich die gegebenen Platzverhältnisse im Hinblick auf eine möglichst einfache und schmerzfreie Einführung des Katheters sehr wesentlich, was speziell im Zusammenhang mit der oben insbesonders angesprochenen Prostatabehandlung nur relativ geringe Katheteraußendurchmesser von vorzugsweise kleiner 5 mm zuläßt, da ansonsten die Einführung über den Harnleiter des Patienten stark behindert bzw. unmöglich wird.

Erschwert werden die angesprochenen Dimensionsprobleme auch noch dadurch, daß zusätzlich zur Mikrowellenantenne zumeist noch ein oder mehrere Thermoelemente zur Messung der aktuellen Kathetertemperatur und ein Kühlsystem in den Katheter eingebaut werden, was den für die Mikrowellenantenne zur Verfügung stehenden Platz weiter einschränkt.

Anordnungen der eingangs angesprochenen Art sind beispielsweise aus der EP-A-370 890 bzw. EP-A-459 535 bekannt, aus welchen Schriften auch die üblicherweise sonst für derartige medizinische Behandlungen erforderlichen bzw. eingesetzten Aggregate sowie das allgemeine Prinzip dieser Behandlung hervorgehen. Als Mikrowellenantenne werden dabei stets stabförmige Strahler ohne Gegengewicht vorgeschlagen.

Weiters ist beispielsweise aus der EP-A1-462 302 die Verwendung einer Spulenantenne als Mikrowellenantenne für den angesprochenen Einsatzzweck bekannt. Auch in der Patentanmeldung FR-A-2 398 510 wird neben einem resonanten Schwingkreis eine reine Spulenantenne propagiert, ohne jedoch auf die Problematik der Anpassung an den Generator einzugehen. Auch im Patent US-A-4 825 880 wird bei der dort genannten Einfachwendelantenne mit einseitigem Anschluß ein Anpassungsgerät verwendet. In der Patentanmeldung WO-A1-92/10932 wird eine Einfachwendelantenne mit beidseitigem Anschluß an ein Koaxialkabel genannt. Bei dieser Antenne wird die rücklaufende Leistung durch einen Mikrowellenzirkulator vom Verstärker ferngehalten und in einem Abschlußwiderstand vernichtet. Auf die Problematik der Anpassung ist auch dort nicht näher eingegangen.

In all diesen Fällen, sowie auch bei den oben angesprochenen stabförmigen Strahlern, läßt sich also der Strahlungswiderstand der Antennen nicht bzw. nur schlecht an den Wellenwiderstand der Anschlußleitung (meist ein Koaxialkabel) anpassen, wodurch Stehwellen und durch die Unsymmetrie der Einspeisung auch Mantelwellen entstehen, was zu einer Abstrahlung eines nennenswerten Teiles der Mikrowellenenergie entlang der Anschlußleitung und damit zu einer unerwünschten und schmerzhaften Erwärmung von gesundem Gewebe entlang der Anschlußleitung führt. Weiters ist in allen Fällen die Antennenlänge im wesentlichen durch die gewählte Mikrowellenfrequenz vorgegeben und kann beispielsweise nicht - wie an sich erwünscht wäre - beliebig an die Größe der zu behandelnden Prostata angepaßt werden.

Aufgabe der Erfindung ist es, die angeführten Nachteile der bekannten Anordnungen der eingangs genannten Art zu vermeiden und insbesonders einen Katheter zur Hyperthermie-Behandlung von lebendem Gewebe so auszubilden, daß eine unbotmäßige Erwärmung der Anschlußleitung verhindert wird und eine unproblematische Anpassung der geometrischen Antennenlänge vorgenommen werden kann.

Dies wird gemäß der Erfindung dadurch erreicht, daß die Mikrowellenantenne als Faltdipol ausgebildet ist, welcher in Form einer Doppelwendel isoliert über einen im wesentlichen zylindrischen, leitfähigen Tragekörper gewickelt ist.

Die Erfindung geht damit einerseits davon aus, daß einer möglichst guten Resonanz-Abstimmung der Mikrowellenantennen im zu behandelnden Gewebe erhöhte Aufmerksamkeit zu schenken ist, da nur so eine möglichst effiziente Erwärmung des zu behandelnden Gewebes unter möglichst weitgehender Schonung des anschließenden gesunden Gewebes möglich ist. Da der Strahlungswiderstand von Antennen, in deren Nahfeld sich leitfähige Materialien befinden, bekanntermaßen schnell absinkt, wobei durch die auftretenden Kapazitäten eine Verstimmung der Resonanzfrequenz auftritt, funktioniert eine - gemäß bisher bekannten Anordnungen - außerhalb des Patienten bzw. bei der Entwicklung abgestimmte Antenne im Körperinneren nicht bzw. eben nur schlecht, wobei der Grad der Verstimmung von der jeweils nicht genau bekannten Leitfähigkeit der Antennenumgebung im Körper bestimmt wird. Durch die erfindungsgemäße Konstruktion der Mikrowellenantenne als Faltdipol wird einerseits ein ausreichend hoher Eingangswiderstand erzielt, wobei weiters durch die Verwendung des leitfähigen Tragekörpers als Wickelkörper für die Doppelwendel eine definierte Leitfähigkeit der unmittelbaren Antennenumgebung erreicht wird, so daß durch vergleichsweise kleine Änderungen der Leitfähigkeit der Gewebeumgebung im Patienten die Verschiebung der Resonanz vernachlässigbar wird. Ein weiterer Vorteil der erfindungsgemäßen Ausbildung ist, daß Mantelwellen, die auf eingangs beschriebene Weise zu einer unerwünschten Erwärmung der Anschlußleitung führen, stark unterdrückt werden, da der zum Mikrowellengenerator zeigende Teil des Faltdipols als Gegengewicht wirkt.

In bevorzugter Ausgestaltung der Erfindung kann bei einem Katheter, bei dem die Anschlußleitung als Koaxialkabel ausgebildet ist, der Endbereich des Koaxialkabels unmittelbar selbst als Tragekörper für die Doppelwendel dienen, was einerseits eine sehr einfache Herstellung mit möglichst geringen Dimensionserhöhungen durch das Aufwickeln der Mikrowellenantenne und andererseits eine große Flexibilität des Katheters in diesem Bereich ergibt, was das Einführen beispielsweise zur Hyperthermie-Behandlung über den Harnleiter des Patienten sehr erleichtert.

Der Einspeisepunkt der Anschlußleitung für die Mikrowellenantenne kann durch eine andere bevorzugte Ausgestaltung der Erfindung aus der Mitte der Doppelwendel verschoben sein, so daß der Eingangswiderstand der Mikrowellenantenne dem Wellenwiderstand der Anschlußleitung entspricht. Dies ermöglicht eine sehr einfache Anpassung, wodurch Stehwellen und Mantelwellen verringert bzw. hintangehalten werden.

Die geometrische Länge der Mikrowellenantenne kann nach einer vorteilhaften anderen Ausbildung der Erfindung über die Wahl der Steigung der Doppelwendel auswähl- bzw. einstellbar sein, was eine einfache und unproblematische Anpassung an die Länge der jeweils zu erwärmenden Gewebezone (im Falle der Prostata etwa 3 bis 7 cm) erlaubt.

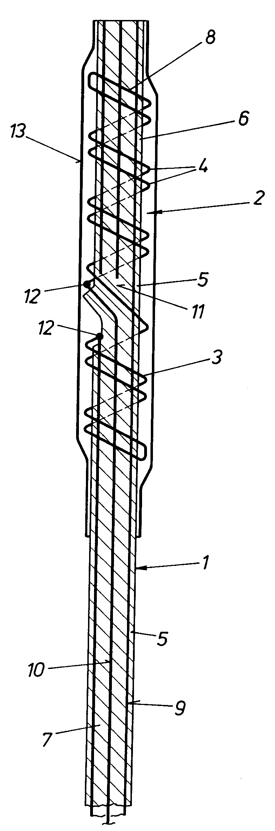

Die Erfindung wird im folgenden noch anhand des in der Zeichnung schematisch dargestellten Schnittbildes eines erfindungsgemäßen Katheters näher erläutert.

Der dargestellte Katheter dient zur Hyperthermie-Behandlung von lebendem Gewebe, insbesondere der hier nicht weiter dargestellten Prostata und weist eine über eine Anschlußleitung 1 von außen angespeiste Mikrowellenantenne 2 auf, die entsprechende Strahlung abgibt, welche im Gewebe auf bekannte Weise in Wärme umgesetzt wird. Die Mikrowellenantenne 2 ist als Faltdipol 3 ausgebildet, welcher in Form einer Doppelwendel 4 unter Zwischenlage einer Isolation 5 über einen im wesentlich zylindrischen, leitfähigen Tragekörper 6 gewickelt ist.

Die Anschlußleitung 1 ist als Koaxialkabel 7 ausgebildet, dessen Endbereich 8 unmittelbar selbst mittels der leitenden Abschirmung 9 den Trageköper 6 für die Doppelwendel 4 bildet.

Der Innenleiter 10 des Koaxialkabels 7 ist an einer Stelle 11 aufgetrennt und über einen Lötpunkt 12 mit dem Faltdipol 3 bzw. der Doppelwendel 4 verbunden. Ein weiterer Lötpunkt 12 verbindet die Abschirmung des Koaxialkabels in diesem Bereich mit dem anderen Ende der Doppelwendel 4, wobei der über die Lötpunkte 12 definierte Einspeisepunkt bzw. -ort der Anschlußleitung 1 aus der Mitte der Doppelwendel 4 in der Darstellung nach unten verschoben ist. Damit wird eine Anpassung des Eingangswiderstandes der Mikrowellenantenne 2 an den Wellenwiderstand der Anschlußleitung 1 ermöglicht. Der unterhalb der Einspeisung gelegene - in Richtung zum nicht dargestellten Mikrowellengenerator zeigende - Teil der Mikrowellenantenne 2 wirkt dabei als Gegengewicht, was Mantelwellen stark unterdrückt.

Durch Variation der Steigung der Doppelwendel 4 kann die geometrische Länge der Mikrowellenantenne 2 leicht variiert werden, so daß sich bei ansonsten im wesentlichen unveränderten Verhältnissen eine Anpassung an die Länge des tatsächlich mit der Mikrowellenantenne 2 zu erwärmenden Bereiches leicht bewerkstelligen läßt.

Als äußere Hülle ist hier im Bereich der Doppelwendel 4 ein Schrumpfschlauch 13 aufgezogen, womit sich insgesamt ein im maximalen Durchmesser extrem klein zu haltender Katheter ergibt - typischerweise kann der Durchmesser im Bereich des Schrumpfschlauches 13 etwa 2,5 mm betragen, was ein sehr leichtes Einführen des Katheters über den Harnleiter des Patienten ermöglicht.

Zur Vereinfachung nicht dargestellt sind hier unter Umständen vorhandene weitere Anordnungen bzw. Aggregate im Bereich der Mikrowellenantenne 2, wie etwa Thermoelemente bzw. Kühleinrichtungen, die unzulässige Erwärmungen im Bereich der Mikrowellenantenne 2 verhindern sollen.

## Patentansprüche

1. Katheter zur Hyperthermie-Behandlung von lebendem Gewebe, insbesonders der Prostata, mit einer über eine Anschlußleitung von außen angespeisten Mikrowellenantenne, **dadurch gekennzeichnet**, daß die Mikrowellenantenne (2) als Faltdipol (3) ausgebildet ist, welcher in Form einer Doppelwendel (4) isoliert über einen im wesentlichen zylindrischen, leitfähigen Tragekörper (6) gewickelt ist.

2. Katheter nach Anspruch 1, wobei die Anschlußleitung als Koaxialkabel ausgebildet ist, dadurch gekennzeichnet, daß der Endbereich (8) der Abschirmung (9) des Koaxialkabels (7) unmittelbar selbst als Tragekörper (6) dient.

3. Katheter nach Anspruch 1 oder 2, dadurch gekennzeichnet, daß der Einspeisepunkt der Anschlußleitung (1) aus der Mitte der Doppelwendel (4) verschoben ist, so daß der Eingangswiderstand der Mikrowellenantenne (2) dem Wellenwiderstand der Anschlußleitung (1) entspricht.

4. Katheter nach einem der Ansprüche 1 bis 3, dadurch gekennzeichnet, daß die geometrische Länge der Mikrowellenantenne (2) über die Wahl der Steigung der Doppelwendel (4) auswähl- bzw. einstellbar ist.
